# EUROPEAN PATENT SPECIFICATION

(11) **EP 4 478 841 B1**
(45) Date of publication and mention of the grant of the patent: **19.11.2025**
(21) Application number: 22710637.4
(22) Date of filing: 07.02.2022
(51) Int. Cl.: H05H 1/48

(54) **COLD ATMOSPHERIC PLASMA GENERATOR AND RESPIRATORY EQUIPMENT FOR THE STIMULATION OF CELLULAR REGENERATION FOR LIVING BEINGS**
KALTER ATMOSPHÄRISCHER PLASMAGENERATOR UND ATEMGERÄT ZUR STIMULATION DER ZELLREGENERATION FÜR LEBEWESEN
GÉNÉRATEUR DE PLASMA ATMOSPHÉRIQUE FROID ET APPAREIL RESPIRATOIRE POUR LA STIMULATION DE LA RÉGÉNÉRATION CELLULAIRE POUR LES ÊTRES VIVANTS

(43) Date of publication of application: 18.12.2024
(73) Proprietor: Bioengineering for the World Corporation S.L., 28230 Las Rozas de Madrid Madrid (ES)
(72) Inventor: CORTÁZAR PÉREZ, Osvaldo Daniel, 28230 LAS ROZAS DE MADRID (Madrid) (ES); LLANA GARCÍA, Pedro Luís, 28230 LAS ROZAS DE MADRID (Madrid) (ES); LOREDO FERNÁNDEZ, Alejandro, 28230 LAS ROZAS DE MADRID (Madrid) (ES); MEGÍA MACÍAS, Ana María, 28230 LAS ROZAS DE MADRID ( Madrid) (ES)
(74) Representative: Herrero & Asociados, S.L.
(86) International application number: PCT/ES2022/070055
(87) International publication number: WO 2023/148412

(56) References cited:
- WO-A1-2021/229233
- WO-A1-2022/173094
- JP-A- 2009 030 608
- KR-B1- 102 253 706
- RU-C2- 2 346 084
- US-A1- 2002 000 779
- LIN Z-H ET AL: "Ar/O2 Argon-Based Round Atmospheric-Pressure Plasma Jet on Sterilizing Bacteria and Endospores", IEEE TRANSACTIONS ON PLASMA SCIENCE, IEEE SERVICE CENTER, PISCATAWAY, NJ, US, vol. 44, no. 12, 1 December 2016 (2016-12-01), pages 3140 - 3147, XP011636131, ISSN: 0093-3813, [retrieved on 20161209], DOI: 10.1109/TPS.2016.2601940
- LAROUSSI M ET AL: "Perspective: The physics, diagnostics, and applications of atmospheric pressure low temperature plasma sources used in plasma medicine", JOURNAL OF APPLIED PHYSICS, AMERICAN INSTITUTE OF PHYSICS, 2 HUNTINGTON QUADRANGLE, MELVILLE, NY 11747, vol. 122, no. 2, 13 July 2017 (2017-07-13), XP012220587, ISSN: 0021-8979, [retrieved on 20170713], DOI: 10.1063/1.4993710
- LU X ET AL: "Reactive species in non-equilibrium atmospheric-pressure plasmas: Generation, transport, and biological effects", PHYSICS REPORTS, ELSEVIER, AMSTERDAM, NL, vol. 630, 1 April 2016 (2016-04-01), pages 1 - 84, XP029523648, ISSN: 0370-1573, DOI: 10.1016/J.PHYSREP.2016.03.003
- LIU CHIH-TUNG ET AL: "Production Enhancement of Reactive Oxygen and Nitrogen Species at Interface of Helium Plasma Jet and Agar", IEEE TRANSACTIONS ON PLASMA SCIENCE, IEEE SERVICE CENTER, PISCATAWAY, NJ, US, vol. 44, no. 12, 1 December 2016 (2016-12-01), pages 3112 - 3116, XP011636125, ISSN: 0093-3813, [retrieved on 20161209], DOI: 10.1109/TPS.2016.2599024
- K-D WELTMANN ET AL: "Plasma medicine-current state of research and medical application", PLASMA PHYSICS AND CONTROLLED FUSION, IOP, BRISTOL, GB, vol. 59, no. 1, 2 November 2016 (2016-11-02), pages 14031, XP020311851, ISSN: 0741-3335, [retrieved on 20161102], DOI: 10.1088/0741-3335/59/1/014031

## Description

### Object of the invention

The object of the present invention relates to a cold atmospheric plasma generator and to respiratory equipment that incorporates the cold atmospheric plasma generator.

### Background of the invention

In physics and engineering, plasma is defined as the fourth state of matter. This state is achieved by transferring enough energy to a gas so that a significant portion of its molecules become ionized. Examples of plasmas in nature are of interest, such as those of electrical discharges in storms and those of the aurora borealis or australis. In addition, there are plasmas that are used technologically, fluorescent and neon lighting tubes are a classic example along with plasma screens. Plasma is also used to cut materials as in the case of plasma torches that use its high temperature and great ability to transfer heat. However, in the last twenty years, devices capable of creating gas plasmas at low temperature atmospheric pressure have been developed, some of which are compatible with living tissue. The key for these plasmas to not damage tissues is the fact that they are out of thermodynamic equilibrium. This characteristic strongly limits the ability to transfer heat to other bodies with which they come into contact, receiving the name of cold atmospheric plasmas or CAP from its acronym. This type of plasma produces a certain concentration of activated oxygen and nitrogen molecules that are called: Reactive Oxygen and Nitrogen Species or "RONS" from its acronym. The importance of RONS is due to its disinfectant and regenerative properties on mammalian cells. This has given rise to the development of a new health branch called plasma medicine that is in full growth phase and within which this invention fits.

CAPs are capable of annihilating bacteria, fungi and viruses with hardly any negative effects on the healthy cells of an organism, as evidenced by the overwhelming number of international publications on the subject. There is a broad international consensus about the scientific basis behind the clinical effects observed when using CAPs. RONS have been shown to be responsible for these effects by virtue of their ability to produce oxidative stress on cells and microorganisms to which cold atmospheric plasma is applied. The difference between the tolerance levels of oxidative stress between a healthy cell and harmful microorganisms is the reason for the above-mentioned selectivity that the CAPs have. Due to structural differences between healthy cells and bacteria, fungi and viruses, such as the size and lack of protection of the DNA of the latter, the level of tolerance is of the order of a hundred times greater in favor of healthy cells. The oxidative stress produced by the RONS from cold atmospheric plasma does not damage healthy tissue, but it eliminates harmful microorganisms, including resistant bacteria.

Taking advantage of the disinfection capacity of CAPs, air purification systems have been developed that use them, such as those detailed in patents: US 8,529,830 B2, US7824475B2, US7427313B2, EP2663403B1 and EP3097750B1, among others. These systems incorporate different types of plasma generators to treat the air in order to eliminate microorganisms and thus achieve a certain degree of cleanliness of the air.

The present invention does not intend to delve further into the benefits of clean air in health and the need for purification systems, but rather opens a new field of use for RONS that generates cold atmospheric plasma for therapeutic use based on the hormesis concept.

A number of processes have been identified during which a low or sublethal dose of a stressful agent or stimulus is capable of activating an adaptive response that increases the resistance of a cell or organism to higher intensity stress. This response is defined as hormesis. Normally we are exposed to hormetic agents such as radiation, heat, heavy metals, antibiotics, pro-oxidant substances, or physical exercise wherein food restriction is also included. The hormetic reaction involves the expression of a large number of genes that encode cytoprotective proteins such as chaperones of the type that respond to thermal stress, antioxidant enzymes, growth factors, metallothioneins, among others. When mammalian cells, including human cells, are exposed to low doses of RONS, these change the intensity of expression of the genes involved in the hormetic response in a period of approximately 9 h. The expression of constitutive, growth and proliferative genes decreases, while the expression of protective genes increases, allowing hormetically stimulated cells to be significantly more resistant to future oxidative stress up to 15 and 30 hours after stimulation. Macroscopically, the hormetic response can be compared to an immune response to vaccination, but with the great difference that the former does not keep long-term memory (beyond 30 hours), and to once again have the hormetic effect, the cells or organisms must be re-exposed to the inducing agent.

In the following documents are described examples of apparatus for the production of reactive species suitable for inhalation:
KR10225370681 and
Liu Ching-Tung et al.: "Production Enhancement of Reactive Oxygen and Nitrogen species at Interface of Helium Plasma Jet and Agar", IEEE TRANSACTIONS OF PLASMA SCIENCE, vol.44, no.12, (2016-12-01), pp. 3112-3116.

### Description of the invention

The present invention relates to a respiratory device that produces and projects onto a human or animal patient a laminar air flow containing a specific concentration of reactive oxygen and nitrogen molecules for inhalation. These RONS are generated by a cold atmospheric plasma generator configured to receive air as an input substance and expel RONS as an output substance and that is part of the respiratory device and that uses the hormetic response of humans and animals particularly against very low levels of oxidative stress.

Thus, the proposed invention consists of a respiratory device that produces a hormetic reaction in humans and animals through the inhalation of small doses of RONS dissolved in the air that have been produced by a cold atmospheric plasma generator.

The proposed device forms a clean air generation unit activated by cold atmospheric plasma that is applied to the patient both in a closed room and in a semi-open chamber specifically designed for this purpose.

For a better conceptual understanding of the invention, it can be considered as composed of two main parts whose functions can be integrated and allow obtaining a laminar flow of air that is relatively rich in RONS produced by a cold atmospheric plasma generator.

Cold atmospheric plasma generator: Plasma is a state of matter wherein ionized particles and electrons move at high speeds producing high-energy and highfrequency shocks that favor the production of chemical reactions that would not occur under normal environmental conditions. This characteristic is what allows the formation of RONS from oxygen and nitrogen molecules.

Respiratory device: This device may include the cold atmospheric plasma generator and is responsible for conditioning the air before being additionally activated by cold atmospheric plasma. This device consists of a silent air blower and a HEPA (High-Efficiency Particulate Arrestance) air filter. The assembly works in such a way that microorganisms and particles are eliminated from the air, including the nanoparticles present. The set assembly includes a nozzle design, for the production of a laminar flow of air. The absence of turbulence in the laminar flow is one of the keys to being able to optimally transport the RONS in the air flow.

In a first aspect, the invention relates to an atmospheric cold plasma generator according to claim 1.

In a second aspect, the invention relates to a respiratory device comprising the plasma generator of claim 1.

Further advantageous embodiments are disclosed in the dependent claims.

### Description of the drawings

To complement the description that is being made and in order to help a better understanding of the characteristics of the plasma generator and the respiratory device according to a preferred example of its practical embodiment, an integral part of said description is attached, a set of drawings wherein, by way of illustration and not limitation, the following has been represented:
Figure 1 shows a conceptual scheme of the respiratory device that integrates the cold atmospheric plasma generator according to the present invention and its application to a patient.
Figure 2 shows a cross-sectional side view of the cold atmospheric plasma generator according to the present invention.
Figure 3 shows a perspective cross-sectional section of the cold atmospheric plasma generator according to the present invention.
Figure 4 shows a cross-section of the respiratory device that integrates the cold atmospheric plasma generator, wherein tests and measurements of the proposed system were carried out.
Figure 5 shows the image obtained from measuring the tension and current signals between the electrodes of the cold atmospheric plasma generator.
Figures 6A and 6B show two light emission spectra obtained from the cold atmospheric plasma produced by the cold atmospheric plasma generator by means of an optical fiber inserted inside the ground electrode.

### Preferred embodiment of the invention

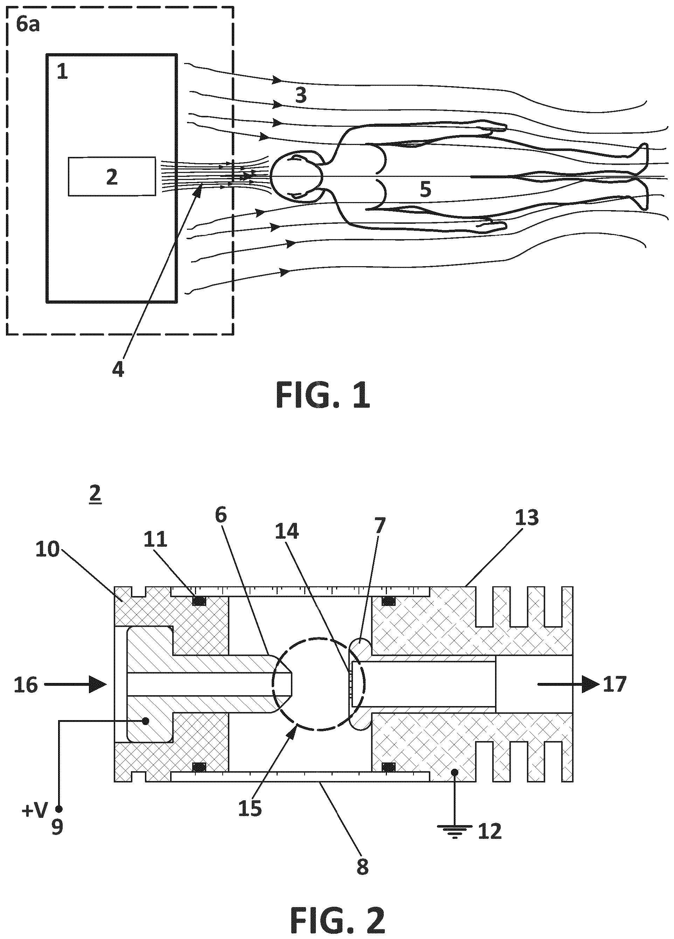

Figure 1 shows a conceptual scheme in the form of a block diagram indicating the location of the respiratory device (1) and the cold atmospheric plasma generator (2). The respiratory device (1) is housed in a box (6a) together with the cold atmospheric plasma generator (2) that injects a plasma-activated air flow (4) into the laminar flow of clean air (3). The total and laminar flow of air is projected on the patient (5).

The laminar flow of clean air (3) is enriched by the injection of the plasma-activated air flow (4) coming from the cold atmospheric plasma generator (2). This laminar flow of air enriched with RONS generated by the cold atmospheric plasma generator (2) is projected onto the patient (5) for inhalation. The entire system is mounted inside the box (6a) and is connected to the home electricity distribution network from wherein it obtains the energy necessary for its operation.

If the air is not properly filtered and treated, it can carry polluting particles of all kinds, including those from the combustion of hydrocarbons inside the cold atmospheric plasma generator (2). In this case, unpredictable chemical reactions could take place with the polluting molecules when they come into contact with the plasma, as well as the compounds that could be generated. Therefore, the use of the previous stage of filtering the air, before being used in the cold atmospheric plasma generator (2), is recommended to allow that the reactive molecules that are generated are only oxygen and nitrogen. This allows unknown compounds not to be introduced into the airflow being applied to the patient.

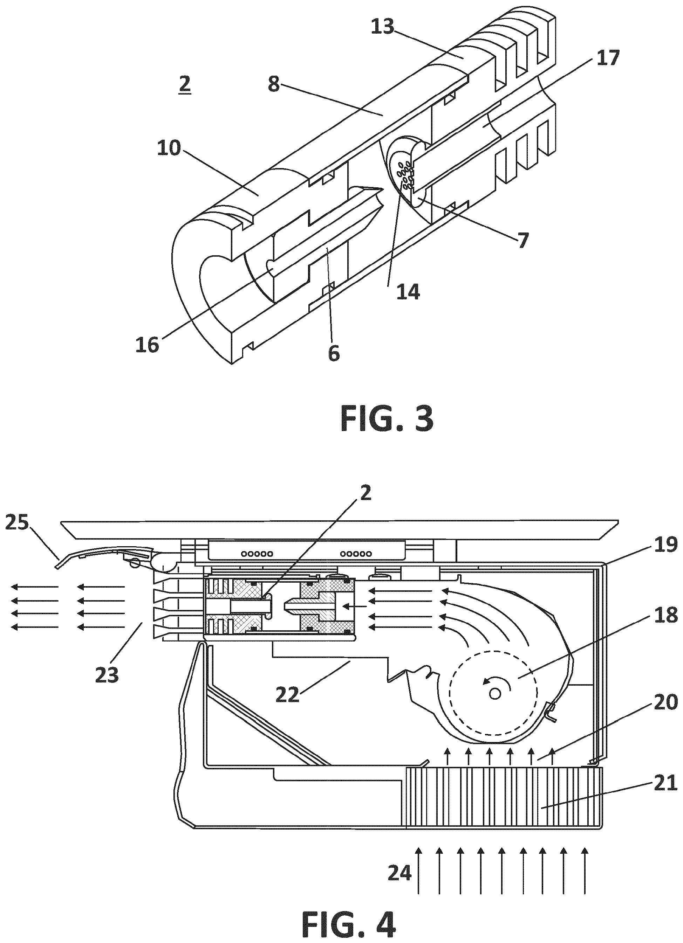

Figures 2 and 3 show cross sections of the cold atmospheric plasma generator (2).

The cold atmospheric plasma generator (2) is made up of two metallic electrodes (6) and (7) facing each other separated by a space (15). The frustoconical positive electrode (6) is connected to a high tension voltage source (9) and the flat metallic electrode (7) is electrically connected to ground (12). Both electrodes have cylindrical symmetry and are held in a coaxial position by two centering pieces, in particular, a supporting piece made of insulating material (10) and a metallic piece (13), respectively. Both centering pieces are kept in a coaxial position by means of a tube of insulating material (8) that is adjusted by means of O-rings (11) to them. The air inlet and outlet are references (16) and (17), respectively.

The positive electrode (6) is connected to a voltage source (9) +V and is held in place by a supporting piece of insulating material (10) that fits with the tube of insulating material (8) by means of an O-ring (11). The positive electrode (6) has a frustoconical shape and has a through hole concentric with its axis. On the other hand, a flat metallic electrode (7) is connected to ground (12) and facing the previous one held by a metallic piece (13) that closes the assembly at the other end of the tube of insulating material (8) with an O-ring. The flat ground electrode (12) has rounded edges and a non-through hole concentric to its axis. This hole or axial perforation leaves a thin wall that forms the front of the ground electrode which, in turn, has a series of through holes (14) of smaller diameter that communicate the interior of the flat metallic electrode (7) with the space (15) that separates the two electrodes as indicated in Figure 2 with a dashed circle.

The air to be activated by means of the cold atmospheric plasma enters the system from the rear part (air inlet (16)) of the positive electrode (6) reaching the space (15) between the electrodes (6) and (7), wherein it circulates and passes through the through holes (14) penetrating inside the flat metallic electrode (7) and finally leaving the system through its outlet (17).

Once an air current is established inside the cold atmospheric plasma generator (2), the voltage is applied to the positive electrode (6) in such a way that its value is high enough to exceed the dielectric capacity of the air in the space (15) between the electrodes (6), (7). In this way a column of cold atmospheric plasma of circulating air is established between the electrodes (6) and (7). Inside the plasma column, reactive oxygen and nitrogen molecules, RONS, are formed, which are dragged by the air current towards the outside through the outlet (17) of the flat metallic electrode (7).

The cold atmospheric plasma generator (2) can inject an air current activated by atmospheric plasma (4) in the main laminar flow that is projected on the patient (5) as shown in Figure 1.

Figure 4 shows a cross section of a diagram showing a preferred embodiment of the respiratory device (1) according to the present invention.

The respiratory device (1) comprises a box (19) made of a material that can be insulating or grounded metal and that contains an air impeller (18) that absorbs air (24) from the outside through an inlet opening (20) wherein an air filter (21) is housed, for example, a HEPA filter, forcing the air flow to pass through it completely. The clean air is projected to the outside through a nozzle (22) that produces a laminar flow. Some of the clean air is forced through the cold atmospheric plasma generator (2) configured to expel RONS. The main air current and the air current from the RONS generator are mixed at the outlet opening (23). A slat (25), preferably made of metallic material, allows directing the air flow to the outlet.

In this embodiment, an air impeller module (18) with cylindrical blades 30 cm long has been used, which is oriented horizontally and is housed in an airtight box made of insulating material. Said box has a rectangular inlet opening (20) oriented downwards wherein the HEPA filter is housed, whose measurements are 30 cm long by 10 cm wide with a thickness of 3 cm. A rectangular nozzle (22) made of sheet metal is installed at the outlet of the air impeller that slightly concentrates the air flow obtained and projects it horizontally forward in the form of a laminar flow air tongue. The set works in such a way that it guarantees that all the projected air has previously passed through the HEPA filter, being free of nanoparticles and other contaminants. The air impeller can operate in four speed regimes which, with the entire system assembled, translate to laminar air flow velocities of 0.40, 0.70, 0.90 and 1.25 m/s measured at 1 m away from the outlet opening (23).

Part of the clean air produced is driven through the cold atmospheric plasma generator (2) which is fixed in the center of the nozzle (22). This portion of air is incorporated into the main flow at the outlet opening (23) corresponding, in this particular case, to 5% of the total. The cross section of Figure 4 is made in the plane wherein the cold atmospheric plasma generator (2) is located. Finally, the thin slat (25) made of metallic material placed horizontally at the outlet can guide the air flow to project it towards a particular direction.

Thus, the air (24) is absorbed by the inlet (20) of the respiratory device (1) due to the depression caused inside the box (19) by the air impeller (18), forcing the incoming flow to pass through the HEPA filter. The air is projected forward forming a laminar flow through the rectangular nozzle (22) wherein part of it is injected into the cold atmospheric plasma generator (2). Finally, the main air flow and the air flow activated by the cold atmospheric plasma generator (2) mix at the outlet opening (23) of the respiratory device (1) projecting onto the patient (5) for inhalation.

The cold atmospheric plasma generator (2) has been built with electrodes (6) and (7) made of brass with a diameter of 10 mm in the space (15) for the formation of the plasma, wherein the inter-electrode separation is 8 mm. The supporting piece of insulating material (10) is made of acetyl resin while the one that supports the flat metallic electrode (7) is made of aluminum. Both have a diameter of 39.9 mm at the height of their respective inserts with O-rings (11). The tube of insulating material is made of quartz with a length of 50 mm and an internal diameter of 40 mm having a wall thickness of 2 mm. The flat metallic electrode (7) has 12 through holes (14) of 1 mm diameter each one distributed in a diameter of 8 mm around its longitudinal axis. A very important aspect regarding the security of the system is that the design of the flat metallic electrode (7) has the advantage of not allowing access to the space (15) for creating the plasma accidentally or intentionally. The small diameter (1 mm) of its through holes (14) does not allow access to the plasma zone, avoiding any risk of electrocution.

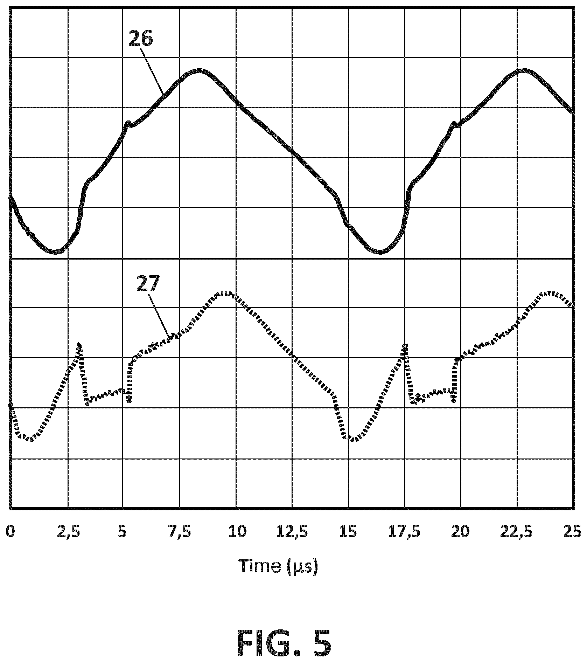

The cold atmospheric plasma generator (2) is powered by a pulsed high tension source at a frequency of 60 kHz which, without load (no load), produces a sinusoidal output tension of 10 kV peak to peak. When the dielectric strength of the air between the electrodes (6), (7) is exceeded, a column of cold atmospheric plasma is established that conducts electricity with a maximum peak current of 60 mA and a tension that is established at 1.82 kV. This working situation, when the cold atmospheric plasma is established, is shown in Figure 5 wherein the tension (26) and current (27) signals measured with an oscilloscope are observed. These curves provide the electrical characterization of the used plasma.

Measurements were made with an oscilloscope and tension and current probes, obtaining values of 60 kHz frequency, 1.82 kV peak-to-peak tension and 60 mA peak current.

In addition, spectroscopic measurements were made in the ultraviolet-visible and ultraviolet range of the light emitted by the plasma. These measurements are very important to check the existence of RONS in the plasma being used and their relative abundance with respect to non-reactive species in air plasmas such as emissions due to single excited neutral atoms and/or molecules. For this, two fiber optic spectrometers were used whose wavelength range is between 200 to 800 nm (UV and Visible) and 200 to 400 nm (UV). The first is used to see the full spectrum and detect emissions that include, in addition to those from RONS, those from neutral atoms and molecules. The second is of higher resolution and allows obtaining emissions only in the UV range where those due to reactive oxygen and nitrogen molecules, RONS, which are of interest for this patent, are found.

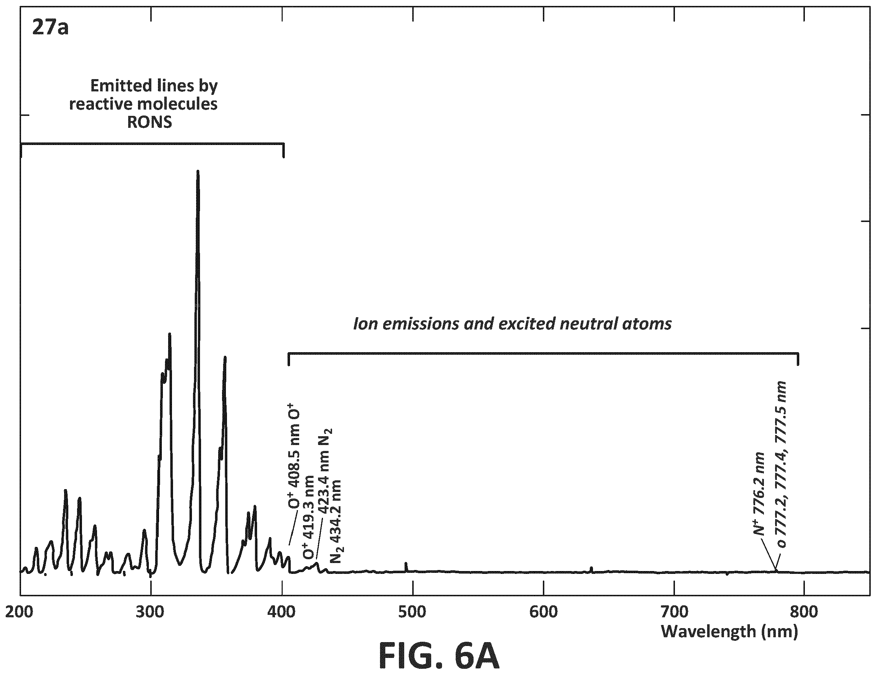

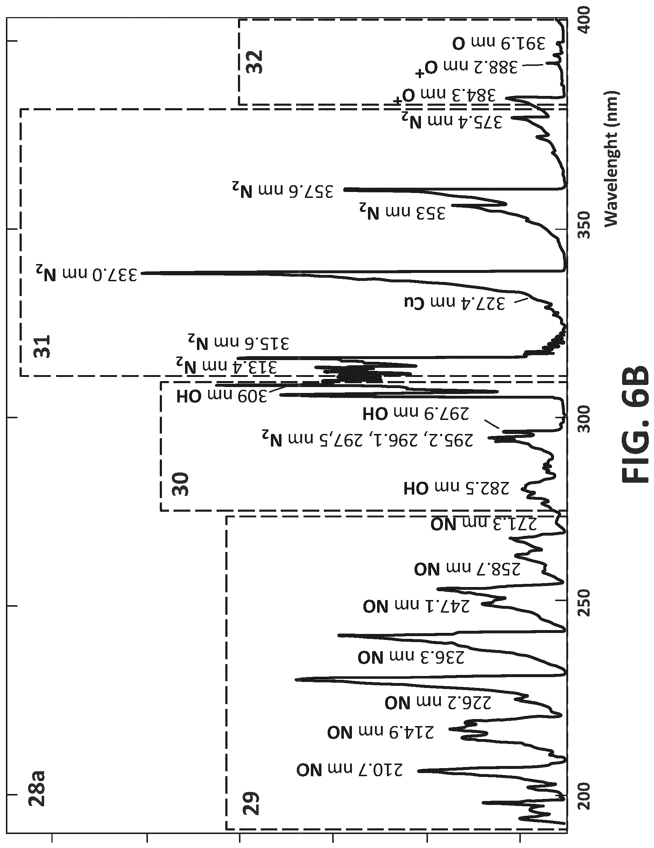

Figures 6A and 6B show the result of the spectral measurements wherein in the image (27a) the complete spectrum is observed in the wavelength range 200 to 800 nm corresponding to UV-VIS. In this spectrum it can be seen that the emission in the visible spectrum (400 to 800 nm) is very low compared to the emission in the ultraviolet range (200 to 400 nm). This abundance in the ultraviolet region implies a relatively high production of RONS. A more detailed spectrum of the UV range is shown in the lower image (28) of Figure 6 wherein the emission between 200 to 400 nm is measured.

The spectrum shows the emission in the wavelength range 200 to 800 nm corresponding to ultraviolet and visible, UV-VIS. In Figure 6B, image (28a) shows the spectrum with emission only in the ultraviolet range, UV, wherein the spectral lines of NO (nitric oxide) (29), OH⁻ (hydroxide ion) (30), N₂ (molecular nitrogen) (31) and neutral and ionized atomic oxygen (32).

The emission of abundant spectral lines is clearly observed, which can be classified into four differential characteristic groups, a first group in the range from 200 to 275 nm corresponding to the emissions of the reactive molecule of nitric oxide NO, a second group in the range from 275 to 310 nm corresponding to the emissions of the reactive molecule of the hydroxide ion OH⁻, a third group of emissions in the range from 310 to 380 nm from the excited neutral molecule of nitrogen N₂ and finally a group of low intensity emissions in the range from 380 to 400 nm from excited neutral atoms and oxygen ions. The cold atmospheric plasma generator has been optimized both in its construction parameters and in those of its electrical supply for the production of RONS, as shown by the measurements carried out. The light radiation analyzed was obtained from inside the flat metallic electrode (7) by introducing an optical fiber from the outlet (17). This allowed the training of light coming from the plasma through the through holes (14).

## Claims

1. Cold atmospheric plasma generator (2) configured to receive air as input substance and expel RONS, Reactive Oxygen and Nitrogen Species, suitable for being inhaled by living beings, as output substance, the cold atmospheric plasma generator (2) comprises:
- a tube of insulating material (8) defining a cavity;
- a supporting piece of insulating material (10) coupled to a first end of the tube of insulating material (8);
- a metallic piece (13) coupled to a second end of the tube of insulating material (8);
- a frustoconical positive electrode (6) established in the cavity of the tube of insulating material (8) and connectable to a voltage source (9);
- a flat metallic electrode (7) established in the cavity of the tube of insulating material (8) and connectable to ground (12),
wherein the electrodes (6, 7) are facing each other defining a space (15) between electrodes (6, 7) in a hollow pin-pepper pot configuration; wherein the supporting piece of insulating material (10) is configured to hold the frustoconical positive electrode (6),
wherein the metallic piece (13) is configured to hold the flat metallic electrode (7),
wherein the frustoconical positive electrode (6) has a through axial perforation configured to allow the passage of air into the cavity of the tube of insulating material (8),
wherein the flat metallic electrode (7) comprises an axial cavity and a plurality of through holes (14) on the surface of the flat metallic electrode (7) that communicate the axial cavity with the cavity of the tube of insulating material (8),
wherein the cold atmospheric plasma generator (2) is configured to:
- establish a circulating air current between the electrodes (6,7);
- electrically connect to ground (12) the flat metallic electrode (7);
- apply a voltage to the frustoconical positive electrode (6) greater than the dielectric strength of the air in the space (15) between the electrodes (6,7);
- generate a column of cold atmospheric plasma from the circulating air between the electrodes (6,7), and
- expel the RONS generated in the cold atmospheric plasma column through the axial cavity of the flat metallic electrode (7) to the outside of the cold atmospheric plasma generator (2).

2. The cold atmospheric plasma generator (2) according to claim 1, wherein the electrodes (6, 7) have cylindrical symmetry and are established in a coaxial position by the supporting pieces of insulating material (10) and the metallic piece (13), respectively.

3. The cold atmospheric plasma generator (2) according to claims 1 or 2, wherein the supporting piece of insulating material (10) is coupled to a first end of the tube of insulating material (8) by means of a first O-ring (11).

4. The cold atmospheric plasma generator (2) according to the preceding claims, wherein the metallic piece (13) is coupled to a second end of the tube of insulating material (8) by means of a second O-ring (11).

5. The cold atmospheric plasma generator (2) according to the preceding claims, wherein the electrodes (6, 7) comprise brass and a diameter of 10 mm and an inter-electrode separation is 8 mm.

6. The cold atmospheric plasma generator (2) according to the preceding claims, wherein the supporting piece of insulating material (10) is made of acetyl resin and with a diameter of 39.9 mm.

7. The cold atmospheric plasma generator (2) according to the preceding claims, wherein the metallic piece (13) comprises aluminum and a diameter of 39.9 mm.

8. The cold atmospheric plasma generator (2) according to the preceding claims, wherein the tube of insulating material (8) is made of quartz with a length of 50 mm, an internal diameter of 40 mm and a wall thickness of 2 mm.

9. The cold atmospheric plasma generator (2) according to the preceding claims, wherein the flat metallic electrode (7) comprises 12 through holes (14) of 1 mm diameter each one distributed in a diameter of 8 mm around the longitudinal axis thereof.

10. A respiratory device (1) comprising an inlet opening (20) and an outlet opening (23), the device comprising:
- an air impeller (18) configured to draw air from the inlet opening (20);
- an air filter (21) configured to generate filtered air driven by the air impeller (18);
- the plasma generator (2) according to claims 1 to 9 configured to absorb part of the filtered air as input substance and expel RONS, Reactive Oxygen and Nitrogen Species, as output substance;
- a nozzle (22) configured to expel the filtered air enriched with RONS through the outlet opening (23) in a laminar flow,
- wherein the laminar flow is apt to be inhaled by living beings.

11. The respiratory device (1) according to claim 10, further comprising an airtight box of insulating material that houses the air impeller (18),
wherein the air impeller (18) comprises cylindrical blades 30 cm long.

12. The respiratory device (1) according to claims 10 or 11, wherein the air filter is a HEPA, High-Efficiency Particulate Arrestance filter whose measurements are 30 cm long by 10 cm wide with a thickness of 3 cm.

13. The respiratory device (1) according to claims 10 to 12, wherein the nozzle (22) has a rectangular shape and is made of sheet metal.

14. The respiratory device (1) according to claims 10 to 13, comprising a slat (25) configured to guide the laminar flow.

15. The respiratory device (1) according to the preceding claim, wherein the slat (25) comprises metal.

## Patentansprüche

1. Kaltatmosphärischer Plasmagenerator (2), der ausgebildet ist, Luft als Eingangssubstanz aufzunehmen und RONS, reaktive Sauerstoff- und Stickstoffspezies, die zum Einatmen durch Lebewesen geeignet sind, als Ausgangssubstanz auszustoßen, wobei der kaltatmosphärische Plasmagenerator (2) aufweist:
- ein Rohr aus isolierendem Material (8), das einen Hohlraum begrenzt;
- ein Halteteil aus isolierendem Material (10), das mit einem ersten Ende des Rohrs aus isolierendem Material (8) verbunden ist;
- ein Metallteil (13), das mit einem zweiten Ende des Rohrs aus isolierendem Material (8) verbunden ist;
- eine kegelstumpfförmige positive Elektrode (6), die in dem Hohlraum des Rohrs aus isolierendem Material (8) angeordnet und mit einer Spannungsquelle (9) verbindbar ist;
- eine flache Metallelektrode (7), die in dem Hohlraum des Rohrs aus isolierendem Material (8) angeordnet und mit Masse (12) verbindbar ist,
wobei die Elektroden (6, 7) einander gegenüberliegen und einen Raum (15) zwischen den Elektroden (6, 7) in einer hohlen Stift-Pfefferstreuer-Konfiguration begrenzen;
wobei das Halteteil aus isolierendem Material (10) ausgebildet ist, die kegelstumpfförmige positive Elektrode (6) zu halten,
wobei das Metallteil (13) ausgebildet ist, die flache Metallelektrode (7) zu halten,
wobei die kegelstumpfförmige positive Elektrode (6) eine durchgehende axiale Perforation aufweist, die ausgebildet ist, den Durchgang von Luft in den Hohlraum des Rohrs aus isolierendem Material (8) zu ermöglichen,
wobei die flache Metallelektrode (7) einen axialen Hohlraum und mehrere Durchgangsbohrungen (14) auf der Oberfläche der flachen Metallelektrode (7) aufweist, die den axialen Hohlraum mit dem Hohlraum des Rohrs aus isolierendem Material (8) verbinden, wobei der kaltatmosphärische Plasmagenerator (2) ausgebildet ist zum:
- Erzeugen eines zirkulierenden Luftstroms zwischen den Elektroden (6, 7);
- elektrisches Verbinden der flachen Metallelektrode (7) mit Masse (12);
- Anlegen einer Spannung an die kegelstumpfförmige positive Elektrode (6), die höher ist als die Durchschlagfestigkeit der Luft in dem Raum (15) zwischen den Elektroden (6, 7);
- Erzeugen einer Säule aus kaltatmosphärischem Plasma aus der zirkulierenden Luft zwischen den Elektroden (6, 7) und
- Ausstoßen der in der Säule aus kaltatmosphärischem Plasma erzeugten RONS durch den axialen Hohlraum der flachen Metallelektrode (7) nach außerhalb des kaltatmosphärischen Plasmagenerators (2).

2. Kaltatmosphärischer Plasmagenerator (2) nach Anspruch 1, wobei die Elektroden (6, 7) Zylindersymmetrie haben und entsprechend durch die Halteteile aus isolierendem Material (10) und das Metallstück (13) in einer koaxialen Position gehalten werden.

3. Kaltatmosphärischer Plasmagenerator (2) nach Anspruch 1 oder 2, wobei das Halteteil aus isolierendem Material (10) mittels eines ersten O-Rings (11) mit einem ersten Ende des Rohrs aus isolierendem Material (8) verbunden ist.

4. Kaltatmosphärischer Plasmagenerator (2) nach den vorstehenden Ansprüchen, wobei das Metallstück (13) mittels eines zweiten O-Rings (11) mit einem zweiten Ende des Rohrs aus isolierendem Material (8) verbunden ist.

5. Kaltatmosphärischer Plasmagenerator (2) nach den vorstehenden Ansprüchen, wobei die Elektroden (6, 7) aus Messing hergestellt sind und einen Durchmesser von 10 mm aufweisen und der Abstand zwischen den Elektroden 8 mm beträgt.

6. Kaltatmosphärischer Plasmagenerator (2) nach den vorstehenden Ansprüchen, wobei das Halteteil aus isolierendem Material (10) aus Acetylharz hergestellt ist und einen Durchmesser von 39,9 mm aufweist.

7. Kaltatmosphärischer Plasmagenerator (2) nach den vorstehenden Ansprüchen, wobei das Metallteil (13) Aluminium aufweist und einen Durchmesser von 39,9 mm hat.

8. Kaltatmosphärischer Plasmagenerator (2) nach den vorstehenden Ansprüchen, wobei das Rohr aus isolierendem Material (8) aus Quarz mit einer Länge von 50 mm, einem Innendurchmesser von 40 mm und einer Wandstärke von 2 mm hergestellt ist.

9. Kaltatmosphärischer Plasmagenerator (2) nach den vorstehenden Ansprüchen, wobei die flache Metallelektrode (7) 12 Durchgangsbohrungen (14) mit einem Durchmesser von jeweils 1 mm aufweist, wovon jede in einem Durchmesser von 8 mm um ihre Längsachse angeordnet ist.

10. Atemgerät (1) mit einer Einlassöffnung (20) und einer Auslassöffnung (23), wobei das Gerät aufweist:
- ein Luftgebläse (18), das ausgebildet ist, Luft aus der Einlassöffnung (20) anzusaugen;
- einen Luftfilter (21), der ausgebildet ist, gefilterte Luft zu erzeugen, die von dem Luftgebläse (18) angetrieben wird;
- den Plasmagenerator (2) nach einem der Ansprüche 1 bis 9, der ausgebildet ist, einen Teil der gefilterten Luft als Eingangssubstanz zu absorbieren und RONS, reaktive Sauerstoff- und Stickstoffspezies, als Ausgangssubstanz auszustoßen;
- eine Düse (22), die ausgebildet ist, die mit RONS angereicherte gefilterte Luft durch die Auslassöffnung (23) in einer laminaren Strömung auszustoßen,
wobei die laminare Strömung von Lebewesen eingeatmet werden kann.

11. Atemgerät (1) nach Anspruch 10, das ferner einen luftdichten Behälter aus isolierendem Material aufweist, in dem das Luftgebläse ( 18) untergebracht ist,
wobei das Luftgebläse (18) zylindrische Flügel mit einer Länge von 30 cm aufweist.

12. Atemgerät (1) nach Anspruch 10 oder 11, wobei der Luftfilter ein HEPA-, Hocheffizienz-Partikel-Arrestanz-, Filter ist, dessen Abmessungen 30 cm Länge, 10 cm Breite und 3 cm Dicke betragen.

13. Atemgerät (1) nach Anspruch 10 bis 12, wobei die Düse (22) eine rechteckige Form aufweist und aus Blech hergestellt ist.

14. Atemgerät (1) nach Anspruch 10 bis 13, das eine Lamelle (25) aufweist, die ausgebildet ist, die laminare Strömung zu führen.

15. Atemgerät (1) nach dem vorstehenden Anspruch, wobei die Lamelle (25) Metall aufweist.

## Revendications

1. Générateur de plasma atmosphérique froid (2) configuré pour recevoir de l'air en tant que substance d'entrée et expulser des RONS, espèces réactives de l'oxygène et de l'azote, adaptées pour être inhalées par des êtres vivants, en tant que substance de sortie, le générateur de plasma atmosphérique froid (2) comprend :
- un tube en matériau isolant (8) définissant une cavité ;
- une pièce de support en matériau isolant (10) couplée à une première extrémité du tube en matériau isolant (8) ;
- une pièce métallique (13) couplée à une deuxième extrémité du tube en matériau isolant (8) ;
- une électrode positive tronconique (6) établie dans la cavité du tube en matériau isolant (8) et pouvant être connectée à une source de tension (9) ;
- une électrode métallique plate (7) établie dans la cavité du tube en matériau isolant (8) et pouvant être connectée à la terre (12),
dans lequel les électrodes (6, 7) sont en vis-à-vis, définissant un espace (15) entre les électrodes (6, 7) dans une configuration de poivrière à broche creuse ;
dans lequel la pièce de support en matériau isolant (10) est configurée pour maintenir l'électrode positive tronconique (6),
dans lequel la pièce métallique (13) est configurée pour maintenir l'électrode métallique plate (7),
dans lequel l'électrode positive tronconique (6) présente une perforation axiale traversante configurée pour permettre le passage d'air dans la cavité du tube en matériau isolant (8),
dans lequel l'électrode métallique plate (7) comprend une cavité axiale et une pluralité de trous traversants (14) sur la surface de l'électrode métallique plate (7) qui font communiquer la cavité axiale avec la cavité du tube en matériau isolant (8),
dans lequel le générateur de plasma atmosphérique froid (2) est configuré pour :
- établir un courant d'air circulant entre les électrodes (6, 7) ;
- connecter électriquement à la terre (12) l'électrode métallique plate (7) ;
- appliquer une tension à l'électrode positive tronconique (6) supérieure à la rigidité diélectrique de l'air dans l'espace (15) entre les électrodes (6, 7) ;
- générer une colonne de plasma atmosphérique froid à partir de l'air circulant entre les électrodes (6, 7), et
- expulser les RONS générées dans la colonne de plasma atmosphérique froid par la cavité axiale de l'électrode métallique plate (7) vers l'extérieur du générateur de plasma atmosphérique froid (2).

2. Générateur de plasma atmosphérique froid (2) selon la revendication 1, dans lequel les électrodes (6, 7) présentent une symétrie cylindrique et sont établies dans une position coaxiale par les pièces de support en matériau isolant (10) et la pièce métallique (13), respectivement.

3. Générateur de plasma atmosphérique froid (2) selon les revendications 1 ou 2, dans lequel la pièce de support en matériau isolant (10) est couplée à une première extrémité du tube en matériau isolant (8) au moyen d'un premier joint torique (11).

4. Générateur de plasma atmosphérique froid (2) selon les revendications précédentes, dans lequel la pièce métallique (13) est couplée à une deuxième extrémité du tube en matériau isolant (8) au moyen d'un deuxième joint torique (11).

5. Générateur de plasma atmosphérique froid (2) selon les revendications précédentes, dans lequel les électrodes (6, 7) comprennent du laiton et un diamètre de 10 mm et une séparation inter-électrodes est de 8 mm.

6. Générateur de plasma atmosphérique froid (2) selon les revendications précédentes, dans lequel la pièce de support en matériau isolant (10) est en résine acétyle et avec un diamètre de 39,9 mm.

7. Générateur de plasma atmosphérique froid (2) selon les revendications précédentes, dans lequel la pièce métallique (13) comprend de l'aluminium et un diamètre de 39,9 mm.

8. Générateur de plasma atmosphérique froid (2) selon les revendications précédentes, dans lequel le tube en matériau isolant (8) est en quartz avec une longueur de 50 mm, un diamètre interne de 40 mm et une épaisseur de paroi de 2 mm.

9. Générateur de plasma atmosphérique froid (2) selon les revendications précédentes, dans lequel l'électrode métallique plate (7) comprend 12 trous traversants (14) de 1 mm de diamètre chacun réparti sur un diamètre de 8 mm autour de son axe longitudinal.

10. Dispositif respiratoire (1) comprenant une ouverture d'entrée (20) et une ouverture de sortie (23), le dispositif comprenant :
- une roue de ventilation (18) configurée pour aspirer de l'air depuis l'ouverture d'entrée (20) ;
- un filtre à air (21) configuré pour générer de l'air filtré propulsé par la roue de ventilation (18) ;
- le générateur de plasma (2) selon les revendications 1 à 9 configuré pour absorber une partie de l'air filtré en tant que substance d'entrée et expulser les RONS, espèces réactives de l'oxygène et de l'azote, en tant que substance de sortie ;
- une buse (22) configurée pour expulser l'air filtré enrichi en RONS par l'ouverture de sortie (23) en un flux laminaire,
- dans lequel le flux laminaire est apte à être inhalé par des êtres vivants.

11. Dispositif respiratoire (1) selon la revendication 10, comprenant en outre un boîtier hermétique en matériau isolant qui abrite la roue de ventilation (18),
dans lequel la roue de ventilation (18) comprend des pales cylindriques de 30 cm de long.

12. Dispositif respiratoire (1) selon les revendications 10 ou 11, dans lequel le filtre à air est un filtre HEPA, haute efficacité pour les particules aériennes, dont les mesures sont de 30 cm de long sur 10 cm de large avec une épaisseur de 3 cm.

13. Dispositif respiratoire (1) selon les revendications 10 à 12, dans lequel la buse (22) présente une forme rectangulaire et est en tôle.

14. Dispositif respiratoire (1) selon les revendications 10 à 13, comprenant un bec (25) configuré pour guider le flux laminaire.

15. Dispositif respiratoire (1) selon la revendication précédente, dans lequel le bec (25) comprend du métal.
